Europäisches Patentamt

European Patent Office

Office européen des brevets

(19)

(11) Numéro de publication : **0 165 208**
**B1**

(12)

# FASCICULE DE BREVET EUROPÉEN

(45) Date de publication du fascicule du brevet :
09.11.88

(21) Numéro de dépôt : **85810225.4**

(22) Date de dépôt : **13.05.85**

(51) Int. Cl.⁴ : **B 29 C 53/00**, A 61 L 15/07,
A 61 F 13/04

(54) Elément thermoformable et utilisation de cet élément.

(30) Priorité : **14.05.84 CH 2368/84**

(43) Date de publication de la demande :
**18.12.85 Bulletin 85/51**

(45) Mention de la délivrance du brevet :
**09.11.88 Bulletin 88/45**

(84) Etats contractants désignés :
**AT BE CH DE FR GB IT LI LU NL SE**

(56) Documents cités :
**EP-A- 0 004 829**
**DE-A- 1 215 859**
**FR-A- 1 570 760**
**GB-A- 1 452 795**
**US-A- 3 326 211**
**US-A- 3 809 600**

(73) Titulaire : **Guignard, Claude**
**Le Vezely/Sergy Gare**
**Saint-Genis-Pouilly (FR)**

**Giglio, Tony**
**15, route des Pilotis**
**CH-1246 Corsier-Port (CH)**

**Benmiloud, Abdelkader**
**55, avenue de Vaudagne**
**CH-1217 Meyrin (CH)**

(72) Inventeur : **Guignard, Claude**
**Le Vezely/Sergy Gare**
**Saint-Genis-Pouilly (FR)**
Inventeur : **Giglio, Tony**
**15, route des Pilotis**
**CH-1246 Corsier-Port (CH)**
Inventeur : **Benmiloud, Abdelkader**
**55, avenue de Vaudagne**
**CH-1217 Meyrin (CH)**

(74) Mandataire : **Dousse, Blasco et al**
**7, route de Drize**
**CH-1227 Carouge/Genève (CH)**

EP 0 165 208 B1

## Description

La présente invention a pour objet un élément thermoformable, comportant deux couches adjacentes de polymères dont l'une au moins est constituée par une mousse élastiquement compressible, au moins l'une de ces couches étant en un polymère thermoplastique et une utilisation de cet élément. Un élément de ce genre est connu du document EP-A-0004829.

Il a déjà été proposé d'utiliser des mousses de polymère thermoplastique, en particulier de polyéthylène, dans le domaine médical, notamment en tant que support orthopédique, pour l'immobilisation de membres fracturés. A cet effet, cette mousse est moulée à chaud autour de la partie du corps à bander. On a également utilisé de telles mousses pour adapter une prothèse à un membre mutilé. A cet effet, la mousse est chauffée à sa température de thermoformage qui se situe autour de 130° et 140 °C et appliquée sur la partie du corps à laquelle on veut l'adapter en la modelant jusqu'à ce qu'elle épouse la forme de cette partie du corps.

D'autres applications de cette mousse ont été proposées, notamment par le EP-A2-0 004 829, dans lequel la mousse est associée à un corps de chauffe électrique afin de pouvoir mouler cette mousse dont a réalisé au préalable une ébauche destinée à adapter étroitement une enveloppe non extensible, notamment rigide, à une partie du corps humain.

Si l'association d'un corps de chauffe électrique est compatible avec un article industriel qui, par définition, est fabriqué en grande série avec les mêmes dimensions, cette solution est difficilement adaptable à un usage médical, en particulier orthopédique, dans la mesure où chaque bandage ne doit pas seulement correspondre à la taille de la personne à soigner, mais également à la ou aux fractures, ainsi qu'à l'emplacement de celles-ci. Etant donné ces contraintes, il est impensable, en pratique, de réaliser des corps de chauffe électriques dimensionnés en fonction de la taille de chaque bandage, étant donné que cette solution conduirait à une multiplicité de corps de chauffe et de les conformer de manière à produire un chauffage homogène sur toute la surface du bandage, ce qui se révèle extrêmement difficile à réaliser et doit être étudié de cas en cas. Cette condition est d'une extrême importance pour le thermoformage étant donné que de telles mousses ont une conductivité thermique très mauvaise, de sorte que la densité de courant par section du corps de chauffe doit être aussi constante que possible pour chaque portion de la surface de mousse à thermoformer.

C'est la raison pour laquelle on a proposé de réaliser des supports orthopédiques avec des matériaux thermoformables sans leur incorporer de moyens de chauffage. L'orthopédiste découpe des morceaux de matériaux thermoformables aux dimensions désirées ou achète des éléments vendus, déjà prédécoupés, les chauffe dans un four ou dans un bain d'eau chaude suivant la température de thermoformage du matériau utilisé et le forme ensuite sur le patient. Un tel bandage orthopédique est décrit dans le FR-A-1.570.760. Dans ce cas, on insère une feuille d'un matériau thermoplastique, rigide à la température ambiante, entre deux feuilles d'une mousse en matériau thermoplastique, afin d'assurer le maintien du membre à immobiliser.

On a également proposé, dans le FR-A-2.120.515 une attelle comprenant une coque rigide dont la face interne est revêtue d'une sorte de chambre étanche formée par une enveloppe souple. Un fluide ou un matériau susceptible de durcir peut être injecté dans cette chambre pour immobiliser le membre à l'intérieur de la coque rigide. Une telle solution est complexe à industrialiser tant il faut réaliser de tailles de coques différentes, cette coque présentant des dimensions immuables.

Un autre facteur, qui entre en ligne de compte dans le thermoformage, est la compatibilité de la température de thermoformage avec ce que supporte la peau. Dans le cas de mousses souples, telles que celles utilisées dans le FR-A-1.570.760 par exemple, leur faible densité permet de supporter une température de thermoformage relativement élevée. Par contre, avec des matériaux plus denses, par exemple des matériaux rigides à température ambiante, on ne peut pas dépasser une température de 50° à 60° si on veut pouvoir utiliser la technique de chauffage dans un four et appliquer ces matériaux encore mous autour du membre à immobiliser. Ceci a nécessité la mise au point de matériaux spécifiques à cette application et qui sont très chers, de sorte que leur utilisation en est freinée.

Le but de la présente invention est d'apporter une solution qui permette de garantir un chauffage régulier de toute la surface de mousse à thermoformer, quelle que soit la forme ou la dimension de cette surface.

A cet effet, la présente invention a pour objet un élément thermoformable selon la revendication 1 ainsi qu'une utilisation de cet élément thermoformable à titre de bandage orthopédique.

Les avantages de cette invention sont nombreux. Le fluide de chauffage peut circuler sur toute la surface à chauffer et la chauffer ainsi régulièrement. L'élément peut être par exemple réalisé en bande de grande longueur et coupé à la longueur désirée en fonction du bandage à réaliser. Le fait de chauffer le bandage de l'intérieur permet, en raison de l'important gradient de température à travers les couches de mousse, d'arriver à thermoformer cet élément sans atteindre une température beaucoup plus élevée que celle du corps humain à la surface externe de cet élément destinée à venir en contact avec la peau. De ce fait, il est possible d'utiliser sans autres des polymères thermoplastiques bon marchés vendus dans le commerce. L'espace fermé dans lequel circule le fluide de chauffage peut également être utilisé pour faire circuler un fluide de refroidissement. notamment pour accélérer le durcissement de la mousse après l'opération de thermoformage.

D'autres particularités et avantages apparaîtront encore à la lumière de la description qui va suivre et des dessins annexés qui illustrent, schématiquement et à titre d'exemple, des variantes de l'élément thermoformable objet de la présente invention.

La fig. 1 est une vue en plan d'une première variante.

La fig. 2 est une vue en coupe selon II-II de la fig. 1.

La fig. 3 est une vue en coupe d'un bandage réalisé avec cette variante.

La fig. 4 est une vue en perspective d'une autre variante.

La fig. 5 est une vue en plan d'une autre variante encore.

La fig. 6 est une vue en coupe selon la ligne VI-VI de la fig.

La fig. 7 est une vue en perspective d'une dernière variante.

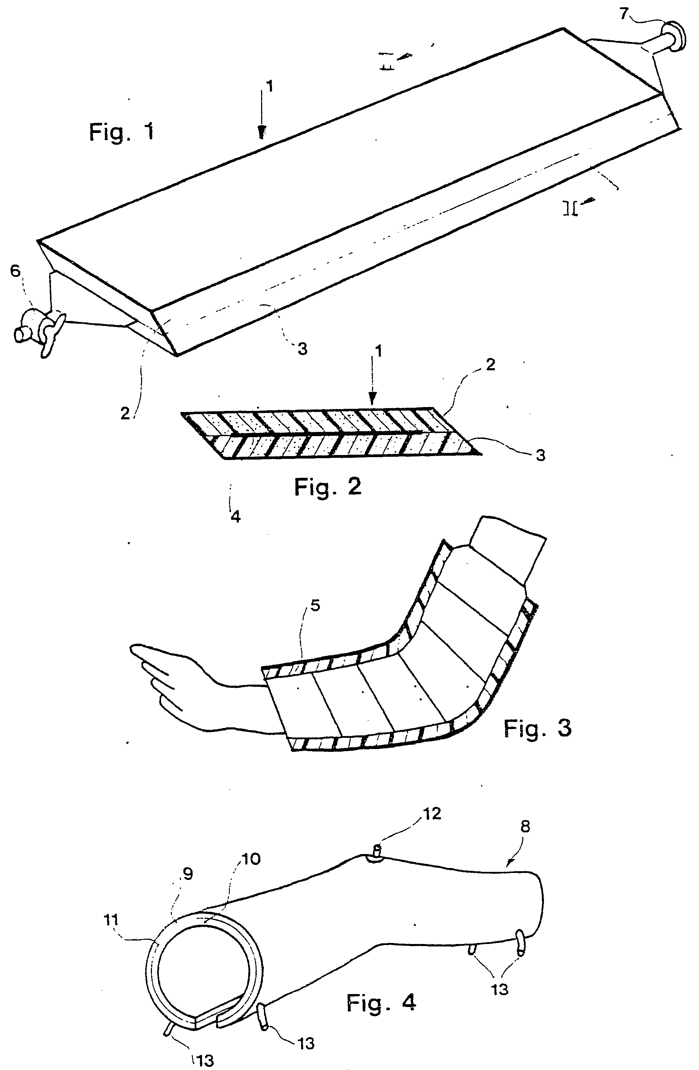

Les figs 1 et 2 illustrent une bande 1 comprenant deux couches de mousse 2 et 3 réalisées en un polymère thermoplastique, notamment en polyéthylène basse densité. Ces couches sont soudées l'une à l'autre le long de leurs deux bords longitudinaux de manière à ménager entre elles un espace en forme de canal 4. Cette opération de soudage peut être réalisée par thermosoudage par ultrasons notamment. On peut également coller les deux bords longitudinaux des couches de mousse 2 et 3 par exemple à l'aide d'une colle du type contact. La fig. 2 montre que, de préférence, la section droite de la bande 1 présente la forme d'un parallélogramme rectangle, de manière à faciliter la jonction entre deux bords longitudinaux adjacents lors de l'enroulement en hélice de la bande 1 autour d'une partie sensiblement cylindrique du corps humain. De préférence, une des faces externes de la bande 1 est recouverte d'une substance auto-collante.

Le canal 4 présente, au repos, la forme d'une simple fente dont les deux lèvres peuvent s'écarter l'une de l'autre lorsqu'un fluide sous pression est envoyé entre les deux couches de mousse comme on l'expliquera par la suite. Cette bande 1 peut être fabriquée en continu et conditionnée sous la forme de rouleaux formés chacun d'une bande de plusieurs mètres de long. Ensuite, celle-ci peut être coupée à la longueur désirée en fonction du bandage à réaliser.

Pour thermoformer le bandage autour du membre fracturé, on fait passer un fluide chaud sous pression à travers le canal 4. Les lèvres de la fente s'écartent pour laisser passer ce fluide. Celui-ci peut être de l'air chaud ou de la vapeur d'eau par exemple dont la température est de l'ordre de 130° à 140 °C qui est la température de thermoformage de la mousse formant les couches 2 et 3. Ce fluide ressort à l'autre extrémité du canal 4. La mousse se ramolit progressivement. Lorsqu'elle est suffisamment ramolie, la température à la surface externe de la bande est de l'ordre de 40 °C. On arrête alors l'envoi du fluide à travers le canal 4 et on forme le bandage en enroulant la bande 1 autour du membre fracturé en serrant suffisamment pour que la bande prenne la forme de cette partie du corps. Ensuite, on laisse refroidir le bandage qui peut alors être recouvert d'un plâtre (fig. 3) ou d'une coquille 5 en matière thermoplastique, qui s'ajuste par thermoformage également autour du bandage. Ces coquilles sont généralement réalisées à partir de plaques de polypropylène.

Etant donné la température de thermoformage du polypropylène qui se situe autour de 200° à 240 °C, le bandage de mousse constitue un isolant empêchant de brûler le patient, de sorte que la coquille peut être directement formée in situ. Toutefois, afin d'empêcher la mousse de polyéthylène de fondre pendant le moulage de la coquille de polypropylène, on peut avantageusement faire passer un fluide de refroidissement à travers le canal 4. Ce fluide sert aussi bien à protéger la mousse que le patient. Ainsi le canal 4 sert aussi à permettre le moulage de la coquille 5 en polypropylène sur le patient, ce qui constitue une simplification appréciable. De telles coquilles ne pouvaient jusqu'ici pas être réalisées in situ en polypropylène en raison de sa température de thermoformage. A cet effet, il fallait recourir à des matériaux du type acrylique tel que celui connu sous la marque déposée orthoplast, dont le prix est beaucoup plus élevé que celui du polypropylène.

Comme on l'a illustré à la fig. 1, après avoir coupé la bande 1 à la longueur désirée, ses deux extrémités peuvent être munies d'une valve anti-retour 6 à l'admission et d'un obturateur 7 à la sortie. Pendant l'opération de chauffage de la bande 1 en vue de son thermoformage, l'obturateur 7 est ouvert. La perte de charge qu'il engendre garantit une bonne répartition du fluide de chauffage dans l'espace en forme de canal 4.

Un certain temps après la formation du bandage réalisé par thermoformage de la bande 1 et pose d'un plâtre ou d'une coquille 5 autour du bandage, les muscles de la partie du corps immobilisée s'atrofient, de sorte qu'un jeu se crée entre cette partie du corps et le bandage. Ce jeu peut être rattrapé en injectant un fluide sous pression dans le canal 4 de la bande 1, après avoir fermé l'obturateur 7. Les lèvres de la fente constituant le canal 4 s'écartent et le jeu est alors supprimé. La pression engendrée ainsi autour de la partie du corps bandée peut d'ailleurs être ajustée par le patient ou par le médecin. Pour garantir une bonne étanchéité de l'espace ménagé entre les couches de mousse, un film étanche tel qu'un film de PVC peut être rapporté sur les faces internes des couches de mousse 2 et 3. On pourrait même envisager la mise en place d'une véritable chambre à air dans le canal 4 à laquelle on fixerait la valve 6 et l'obturateur 7. Cette fixation peut être réalisée par collage ou par soudage à chaud.

L'invention n'est pas limitée à la forme d'exécution dans laquelle l'élément thermoformable se présente sous la forme d'une bande. Comme illustré par la fig. 4, le bandage peut également présenter la forme d'une ébauche d'une partie du corps, par exemple de la jambe et du genou. Le concept reste toutefois identique à celui de la bande. Le bandage 8 de la fig. 4 comporte deux couches de mousse 9 et 10 soudées ou collées entre elles à leurs périphéries respectives et ménageant un espace fermé 11. Dans cette forme d'exécution, le bandage 8 est muni d'une valve anti-retour 12 et de quatre obturateurs 13. Le principe est identique à celui de la bande 1. Un fluide chaud de 130° à 140° est envoyé à travers la valve 12 et ressort par les ouvertures des obturateurs 13. L'ébauche ainsi chauffée à la température de thermoformage est alors appliquée autour de la jambe du patient, en particulier, on prend soin de mouler étroitement des points d'appui sous le genou, au-dessus de la fracture pour permettre d'associer un support (non représenté) au bandage. Comme dans le cas de la forme d'exécution précédente, une coquille rigide en plâtre ou en polypropylène notamment peut être formée autour du bandage 8. Dans le cas de la formation de la coquille en polypropylène in situ, un fluide de refroidissement peut également être envoyé dans l'espace ménagé entre les deux couches de mousse 9 et 10, comme décrit précédemment.

Les mousses de polyéthylène à pores fermés ont la propriété de retrouver leur forme initiale lorsqu'elles sont réchauffées à leur température de thermoformage, de sorte que le bandage peut être thermoforme plusieurs fois de suite au cas où il s'avèrerait nécessaire de modifier la position d'immobilisation des membres.

La description qui précède montre l'extrême souplesse d'utilisation de l'élément thermoformable objet de l'invention aussi bien dans sa forme en bande que dans sa forme de jambière préformée de la fig. 4. Dans ce dernier cas, il est évident que différentes tailles de jambières peuvent être réalisées industriellement pour permettre une adaptation à l'ensemble de la population. Cet élément thermoformable permet également une simplification du travail de l'orthopédiste dans la mesure où la prise de forme préalable de la jambe ou du bras en vue de réaliser un gabarit de moulage n'est plus nécessaire, tous les moulages, celui du bandage aussi bien que celui de la coquille entourant ce bandage, pouvant être réalisés directement in situ. Le mode de chauffage de l'élément thermoformable évite également tout risque de brûlure. Enfin, l'espace réservé entre les couches de mousse peut être utilisé non seulement pour chauffer, mais pour refroidir et en le fermant à son entrée et à sa ou à ses sorties peut servir à contenir un fluide sous pression pour combler les jeux consécutifs à l'atrophie des muscles. A cet effet, le fluide utilisé peut également être un gel.

Dans le cas où l'élément thermoformable se présente sous la forme d'une ébauche plate ou préformée du type de celle de la fig. 4, de préférence, la coquille rigide constitue directement la couche externe 9 du bandage 8. Avantageusement, cette couche externe 9 rigide à la température ambiante peut être réalisée à partir d'une feuille d'un copolymère d'éthylène-vinyl acétate de 3,2 mm d'épaisseur tel que celui vendu sous la dénomination commerciale « Worblex Electra 7013 » par Gurit-Worbla AG à Ittigen dans le canton de Berne (Suisse). Ce produit présente une densité de 1,16 kg/dm$^3$ à 23 °C et un point de ramollissement VICAT à 73 °C. La mousse qui lui est associée en tant que couche interne 10 est constituée par une feuille de polyéthylène réticulé par irradiation de 4 mm d'épaisseur de 67 kg/m$^3$ de densité et dont la température de thermoformage est située autour de 120 °C. Cette mousse est notamment vendue sous la marque Alveolit 1500 par Alveo AG à Lucerne (Suisse).

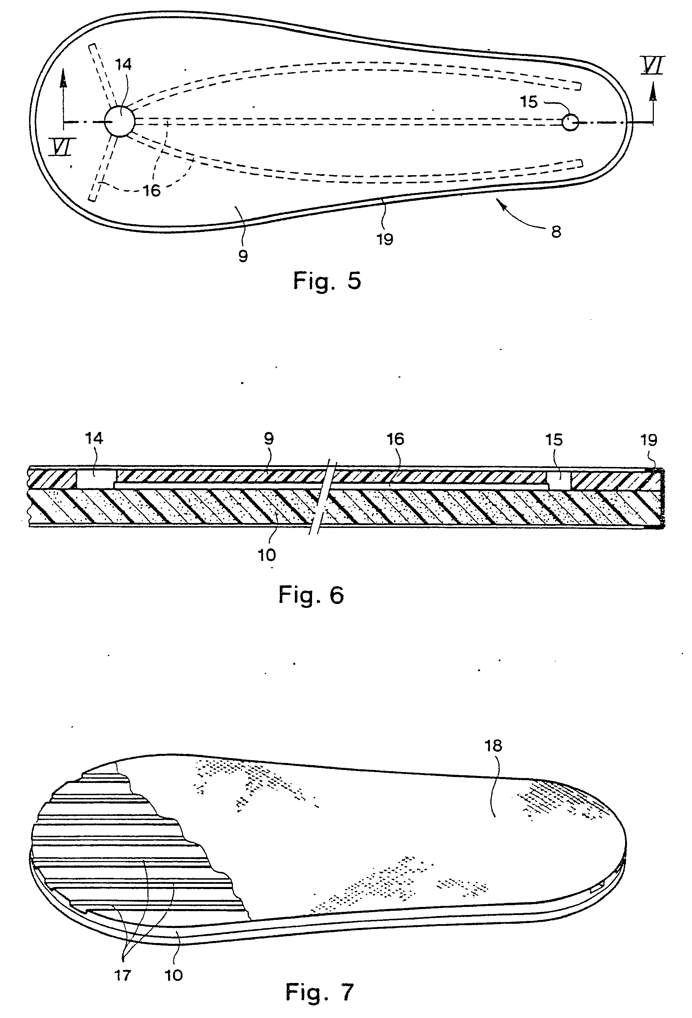

Dans un tel cas où les deux couches 9 et 10 sont en des matériaux qui ne sont pas susceptibles de se souder à chaud, les bords des couches adjacentes sont fixés l'un à l'autre par collage à l'aide d'une colle contact, par couture par une bande adhésive 19 (fig. 6) notamment. Si les deux couches sont en un même matériau ou en deux matériaux susceptibles de se souder à leur température de ramollissement, les bords des couches peuvent être soudés comme on l'a déjà dit. Toutefois, avec de tels matériaux soudables à chaud, il est alors nécessaire, si on veut pouvoir thermoformer l'élément plusieurs fois, de répartir du talc ou de la magnésie à l'intérieur de l'espace fermé délimité entre ces deux couches pour empêcher leur collage au moment du thermoformage.

Dans le cas de surfaces relativement étendues, il est nécessaire de prendre des mesures pour assurer une irrigation régulière de tout l'espace par le fluide de chauffage. Comme illustré par les figures 5 à 7, ces mesures consistent premièrement à ménager une ouverture d'admission 14 à proximité d'un bord de l'espace fermé délimité entre les couches adjacentes 9 et 10, dont la section est sensiblement supérieure à celle de la ou des ouvertures d'évacuation 15 de manière à créer une perte de charge. La seconde mesure consiste à ménager des canaux d'irrigation 16 dans l'épaisseur de l'une des couches reliant l'ouverture d'admission 14 à la ou aux ouvertures d'évacuation 15. Ces canaux 16 ont, dans cet exemple, une section de l'ordre de 1 mm$^2$. Ils ont pour but de répartir le fluide de chauffage sous pression sur toute la surface de l'espace fermé. Etant donné que la perte de charge est créée à la sortie de ce fluide sous pression, les couches adjacentes 9 et 10, dont l'une au moins est déformable élastiquement, sont écartées l'une de l'autre de sorte que le fluide se répartit entre les canaux et irrigue ainsi tout l'espace en provoquant un chauffage régulier des couches adjacentes 9 et 10 à leur température de thermoformage. Il n'est pas nécessaire que chaque canal d'irrigation 16 aboutisse à une ouverture d'évacuation, il est également utile de ménager des canaux d'irrigation intermédiare 16a qui n'aboutissent directement à aucune sortie. L'écartement entre ces canaux d'irrigation 16 ou 16a est de l'ordre de 10 cm.

Dans le cas où il s'agit de développer une grande surface, par exemple dans le cas d'un corset, il peut être avantageux de prévoir une ventilation de la surface de mousse en contact avec la peau soit directement soit de préférence avec interposition d'un tricot 18. Comme illustré par la fig. 7, cette ventilation est réalisée grâce à des canaux 17 ménagés dans l'épaisseur de la couche interne 10 et orientés de manière à occuper une position sensiblement verticale afin d'assurer une circulation d'air par effet de cheminée. La présence d'un tricot 18 entre la mousse 10 et la peau permet d'éviter ou d'atténuer l'effet de succion exercé sur la peau un peu à l'instar d'une ventouse. Ceci permet notamment de ménager des canaux 17 de section suffisante pour assurer une bonne circulation d'air. Des canaux de 3 × 3 mm donnent de bons résultats. Si la présence d'un tricot n'est pas prévue, il serait bien de réduire la section des canaux à environ 1 × 1 mm.

Selon une variante non représentée, au lieu d'utiliser deux couches soudées, collées, cousues ensemble ou fixées par une bande adhésive 19 (fig. 6), on pourrait envisager de former ces couches par écrasement d'un tube de mousse extrudé. Dans un tel cas, les deux couches sont nécessairement en mousse d'un même matériau, de sorte qu'il y aura lieu de revêtir l'intérieur de talc pour éviter le collage lors du thermoformage.

Une autre variante peut encore être envisagée dans laquelle la couche extérieure n'est ni constituée par de la mousse souple ni par un matériau rigide à température ambiante, mais par un tricot élastique imprégné de PVC pour le rendre étanche, la couche intérieure étant en mousse d'un matériau thermoformable fixé au tricot selon l'un des modes de fixation susmentionnés pour ménager entre leurs bords un espace fermé. Un tel tricot peut être utilisé par exemple pour comprimer une cheville au moyen d'un laçage, le thermoformage de la mousse permettant de répartir uniformément la pression du tricot élastique.

Comme on l'a dit précédemment, le mode de chauffage de l'élément thermoformable selon l'invention est constitué par un fluide chaud envoyé dans l'espace fermé ménagé entre les deux couches de cet élément. Selon une variante, il est également possible de réaliser le chauffage de produits plats réalisés par exemple à l'aide d'une couche de polyéthylène rigide 9 et d'une couche de mousse de polyéthylène 10 (fig. 5) et à pores fermés en introduisant de l'eau froide dans l'espace fermé ménagé entre ces deux couches. Cette eau peut être introduite en reliant l'ouverture d'admission 4 (figs 5 et 6) à un robinet d'eau courante ou également en trempant l'élément dans une cuvette d'eau. Dans ce dernier cas, la répartition de l'eau peut être améliorée par la présence d'une couche hydrophile, tel que du coton, entre les deux couches de polyéthylène. Ensuite, l'élément est placé dans un four à micro-ondes où l'eau est chauffée, se transforme en vapeur et ressort de l'espace fermé par les ouvertures 14 et 15 en chauffant l'espace fermé et en communiquant de l'intérieur de l'élément la chaleur aux couches 9 et 10, exactement comme le fait la vapeur ou l'air chaud sous pression.

On peut encore préciser que, dans le cas de grandes surfaces, on peut prévoir des points ou lignes de fixation des deux couches à l'intérieur de l'espace fermé afin d'empêcher le glissement d'une couche par rapport à l'autre.

Bien que l'invention ait été décrite en particulier en liaison avec son utilisation en tant que bandage orthopédique, d'autres applications de l'invention sont également considérées comme comprises dans le champ de la protection. C'est ainsi que l'on pourrait envisager l'utilisation d'un tel élément en tant que joint d'étanchéité thermoformable, matelas thermoformable pour brûlés, sièges de voiture etc.

**Revendications**

1. Elément thermoformable comportant deux couches adjacentes (2, 3, 9, 10) de polymères dont l'une au moins est constituée par une mousse élastiquement compressible, au moins l'une de ces couches étant en un polymère thermoplastique, caractérisé par le fait que les bords de ces couches (2, 3, 9, 10) sont fixés de manière étanche l'un à l'autre, ces couches délimitant entre elles un espace ferme relié avec l'extérieur par au moins deux ouvertures (12, 13, 14, 15) dont l'une constitue une admission pour un fluide de chauffage dudit polymère thermoplastique à sa température de thermoformage et dont l'autre constitue une évacuation de ce fluide.

2. Elément selon la revendication 1, caractérisé par le fait que les deux couches (2, 3) sont en une mousse de polymère thermoplastique.

3. Elément selon la revendication 1, caractérisé par le fait que l'une desdites couches (9) est en un matériau thermoplastique rigide à la température ambiante.

4. Elément selon la revendication 1, caractérisé par le fait que lesdites ouvertures d'admission et d'évacuation sont contrôlées respectivement par une valve anti-retour (6) et un obturateur (7).

5. Elément selon la revendication 1, caractérisé par le fait que lesdits bords sont fixés l'un à l'autre par thermo-soudage.

6. Elément selon la revendication 1, caractérisé par le fait que les faces adjacentes desdites couches (2, 3, 9, 10) sont associées à un film étanche.

7. Elément selon la revendication 1, caractérisé par le fait qu'il est réalisé sous la forme d'un ruban (1) formé de deux couches (2, 3) soudées longitudinalement.

8. Elément selon la revendication 1, caractérisé par le fait que l'une de ses faces externes est auto-collante.

9. Elément selon la revendication 1, caractérisé par le fait que l'une desdites couches est en un matériau élastiquement extensible.

10. Elément selon la revendication 1, caractérisé par le fait que la section de l'ouverture d'admission (14) est supérieure à celle de l'ouverture d'évacuation (15).

11. Elément selon la revendication 1, caractérisé par le fait que l'une des faces ménageant ledit espace fermé comporte des canaux d'irrigation (16) partant de l'ouverture d'admission (14) et étant répartis régulièrement sur la surface sur laquelle le fluide de chauffage doit être distribué.

12. Utilisation de l'élément thermoformable selon l'une des revendications précédentes à titre de bandage orthopédique.

13. Utilisation selon la revendication 12, caractérisée par le fait que la face de mousse (10) destinée à venir appliquée directement ou indirectement contre la peau présente des canaux de ventilation (17) orientés pour être sensiblement verticaux.

## Claims

1. A thermoformable element comprising two adjacent layers (2, 3, 9, 10) of polymers one of which at least one consists of an elastically compressible foam, at least one layer being made of a thermoplastic polymer, characterized in that the edges of these layers (2, 3, 9, 10) are imperviously united together so as to define between the layers an enclosed space connected to the outside by at least two openings (12, 13, 14, 15) of which one opening constitutes an inlet for a fluid for heating the said thermoplastic polymer to its thermoforming temperature and the other of which openings constitutes an outlet for this fluid.

2. An element according to claim 1, characterised in that both said layers (2, 3) are made of a thermoplastic polymer foam.

3. An element according to claim 1, characterised in that one of the said layers (9) is made of a thermoplastic material which is rigid at room temperature.

4. An element according to claim 1, further including a non-return valve (6) and a plug (7) respectively controlling the said inlet and outlet openings.

5. An element according to claim 1, characterised in that the said edges are secured to one another by heat welding.

6. An element according to claim 1, further including an impervious film associated with the adjacent faces of the said layers (2, 3, 9, 10).

7. An element according to claim 1, characterised in that it is made in the form of a strip (1) formed from two longitudinally welded layers (2, 3).

8. An element according to claim 1, characterised in that one of the outer faces of the element is self-adhesive.

9. An element according to claim 1, characterised in that one of the said layers is made of an elastically extensible material.

10. An element according to claim 1, characterised in that the cross-section of the inlet opening (14) is greater than that of the oulet opening (15).

11. An element according to claim 1, characterised in that one of the faces forming the said enclosed space is provided with irrigation channels (16), beginning at the inlet opening (14), and distributed evenly across the surface on which the heating fluid is to be distributed.

12. The use of a thermoformable element, according to any one of the preceding claims, as an orthopaedic bandage.

13. The use according to claim 12, characterised in that a foam face for direct or indirect application on the skin which face has ventilation channels (17) positioned to be substantially vertical.

## Patentansprüche

1. Wärmeverformbares Element mit zwei aufeinander liegenden Polymerschichten (2, 3, 9, 10), von denen wenigstens eine aus einem elastischen, zusammendrückbaren Schaum und wenigstens eine Schicht aus einem thermoplastischen Polymer besteht, dadurch gekennzeichnet, daß die Ränder dieser Schichten (2, 3, 9, 10) dicht miteinander verbunden sind und miteinander einen geschlossenen Raum begrenzen, der wenigstens über zwei Öffnungen (12, 13, 14, 15) nach außen verbunden ist, von denen eine einen Eintritt für ein Heizmedium des thermoplastischen Polymers auf seine Wärmeverformungstemperatur und die andere einen Austritt für dieses Medium bilden.

2. Element nach Anspruch 1, dadurch gekennzeichnet, daß die beiden Schichten (2, 3) aus einem thermoplastischen Polymerschaum sind.

3. Element nach Anspruch 1, dadurch gekennzeichnet, daß eine der Schichten (9) aus einem bei Raumtemperatur starren thermoplastischen Material ist.

4. Element nach Anspruch 1, dadurch gekennzeichnet, daß die Eintritts- bzw. Austrittsöffnung durch ein Einwegventil (6) und durch eine Verschlußeinrichtung (7) gesteuert sind.

5. Element nach Anspruch 1, dadurch gekennzeichnet, daß die Ränder miteinander durch Thermoverschweissen verbunden sind.

6. Element nach Anspruch 1, dadurch gekennzeichnet, daß die benachbarten Flächen der Schichten (2, 3, 9, 10) mit einem dichten Film versehen sind.

7. Element nach Anspruch 1, dadurch gekennzeichnet, daß es in Form eines Bandes (1) gegeben ist, das durch zwei in Längsrichtung verschweißten Schichten (2, 3) gebildet wird.

8. Element nach Anspruch 1, dadurch gekennzeichnet, daß eine seiner äußeren Flächen selbstklebend ist.

9. Element nach Anspruch 1, dadurch gekennzeichnet, daß eine der Schichten aus einem elastisch dehnbaren Material ist.

10. Element nach Anspruch 1, dadurch gekennzeichnet, daß der Querschnitt der Eintrittsöffnung (14) größer ist als jener der Austrittsöffnung (15).

11. Element nach Anspruch 1, dadurch gekennzeichnet, daß eine der den geschlossenen Raum bildenden Flächen Verteilerkanäle (16) trägt, die von der Eintrittsöffnung (14) ausgehend und regelmäßig zur Fläche verteilt sind, auf der das Heizmedium verteilt werden soll.

12. Verwendung des wärmeverformbaren Elementes nach einem der vorhergehenden Ansprüche als orthopädische Bandage.

13. Verwendung nach Anspruch 12, dadurch gekennzeichnet, daß die Schaumfläche (10), die direkt oder indirekt auf der Haut aufgebracht werden soll, Belüftungskanäle (17) hat, die im wesentlichen vertikal ausgerichtet sind.

Fig. 1

Fig. 2

Fig. 3

Fig. 4

**Fig. 5**

**Fig. 6**

**Fig. 7**